(19) **Europäisches Patentamt**

**European Patent Office**

**Office européen des brevets**

(11) Veröffentlichungsnummer: **0 137 187 B1**

(12) **EUROPÄISCHE PATENTSCHRIFT**

(45) Veröffentlichungstag der Patentschrift:
**15.07.87**

(21) Anmeldenummer: **84109340.4**

(22) Anmeldetag: **07.08.84**

(51) Int. Cl.⁴: **C 07 D 211/02,** C 07 D 207/06, C 07 D 295/02, C 07 D 223/04 // B01J23/74

(54) Verfahren zur Herstellung von 5- bis 7-gliedrigen cyclischen Iminen.

(30) Priorität: **17.08.83 DE 3329692**

(43) Veröffentlichungstag der Anmeldung:
**17.04.85 Patentblatt 85/16**

(45) Bekanntmachung des Hinweises auf die Patenterteilung:
**15.07.87 Patentblatt 87/29**

(84) Benannte Vertragsstaaten:
**BE CH DE FR GB IT LI NL**

(56) Entgegenhaltungen:
**DE - A - 2 429 293
DE - B - 2 108 455
DE - B - 2 514 004
DE - C - 699 032
DE - C - 1 061 760
US - A - 3 975 392**

(73) Patentinhaber: **BASF Aktiengesellschaft, Carl-Bosch-Strasse 38, D-6700 Ludwigshafen (DE)**

(72) Erfinder: **Frank, Gerhard, Dr., Heidelberger Strasse 11, D-6945 Hirschberg (DE)**
Erfinder: **Neubauer, Gerald, Dr., Mozartstrasse 24, D-6940 Weinheim (DE)**

**Beschreibung**

Aus der DE-AS 2 514 004 ist bekannt, dass man cyclische Imine, wie 3-Methylpiperidin, durch Hydrierung von 2-Methylglutarsäuredinitril in flüssiger Phase unter Mitverwendung von Ammoniak in Gegenwart von Nickelkatalysatoren erhält. Bei diesem Verfahren ist jedoch die Ausbeute an 3-Methylpiperidin noch verbesserungsbedüftig. Darüber hinaus erhält man nach dem bekannten Verfahren erhebliche Mengen an höherkondensierten Polyaminen.

Es war deshalb die technische Aufgabe gestellt, ein Verfahren zur Herstellung von 3- bis 7gliedrigen cyclischen Iminen zur Verfügung zu stellen, das mit hohen Ausbeuten verläuft und bei dem möglichst wenig hochkondensierte Polyamine entstehen und zudem der Katalysator eine lange Lebensdauer hat.

Diese technische Aufgabe wird gelöst in einem Verfahren zur Herstellung von 5- bis 7gliedrigen cyclischen Iminen mit einer Iminogruppe von 4 bis 6 Kohlenstoffatomen im Ring, wobei mindestens ein Kohlenstoffatom einen Alkylrest mit 1 bis 4 Kohlenstoffatomen als Substituenten enthalten kann, durch Hydrieren von $C_2$- bis $C_4$-Alkandinitrilen, die an mindestens einem Kohlenstoffatom einen Alkylrest mit 1 bis 4 Kohlenstoffatomen als Substituenten haben können, bei einer Temperatur von 180 bis 300°C und unter erhöhtem Druck in flüssiger Phase in Gegenwart von Katalysatoren, dadurch gekennzeichnet, dass man Eisenkatalysatoren verwendet, die durch Reduktion von Eisenoxiden mit Wasserstoff bei einer Temperatur $\leq$ 500°C erhalten worden sind.

Das neue Verfahren hat den Vorteil, dass höhere Ausbeuten an cyclischen Iminen erzielt werden, und die Mitverwendung von Ammoniak nicht erforderlich ist. Darüber hinaus hat das neue Verfahren den Vorteil, dass der Anfall an höher kondensierten Polyaminen reduziert wird. Ferner hat das neue Verfahren den Vorteil, dass die verwendeten Katalysatoren eine lange Lebensdauer haben. Das neue Verfahren ist insofern bemerkenswert als bei Temperaturen von über 200°C mit einer vermehrten Bildung an höherkondensierten Polyaminen zu rechnen war.

Als Ausgangsstoffe verwendet man $C_2$- bis $C_4$-Alkandinitrile, die an mindestens einem Kohlenstoffatom einen Alkylrest mit 1 bis 4 Kohlenstoffatomen als Substituenten enthalten können. Bevorzugt sind solche $C_2$- bis $C_4$-Alkandinitrile, die an einem Kohlenstoffatom eine Methyl oder Ethylgruppe als Substituenten haben. Geeignete Ausgangsstoffe sind beispielsweise: Bernsteinsäuredinitril, 2-Ethylbernsteinsäuredinitril, Glutarsäuredinitril, 2-Methylglutarsäuredinitril, Adipinsäuredinitril oder Trimethyladipinsäuredinitril. Besondere Bedeutung hat 2-Methylglutarsäuredinitril erlangt.

Obzwar die Mitverwendung von Ammoniak nicht erforderlich ist, kann für die Umsetzung flüssiger Ammoniak mitverwendet werden. Vorteilhaft wendet man je Volumenteil Dinitril 1 bis 12 Volumenteile Ammoniak an. Die Hydrierung wird mit Wasserstoff bei erhöhter Temperatur durchgeführt. Hierbei hält man Temperaturen von 180 bis 300°C, insbesondere 200 bis 260°C ein. Vorteilhaft wendet man hierbei Drücke von 150 bis 320 bar an. Es versteht sich, dass die Druck- und Temperaturbedingungen so gewählt werden, dass die Hydrierung in flüssiger Phase verläuft. Ferner hat es sich bewährt, wenn man je Volumenteil frisch zugeführtes Dinitril 1 bis 15, insbesondere 6 bis 10 Volumenteile rohes Hydriergemisch zurückführt. Ein solches Hydriergemisch enthält z.B. neben nichtumgesetztem Dinitril, das entsprechende cyclische Imin, Alkandiamin sowie geringe Mengen höherkondensierte Produkte, ein typisches Hydriergemisch enthält z.B. hauptsächlich 3-Methylpiperidin, daneben wenig 2-Methyl-1,5-diaminopentan, geringe Mengen an Methylglutarsäuredinitril sowie weitere nicht näher definierte Amine sowie Ammoniak aus der Cyclisierungsreaktion und ggf. durch Mitverwendung flüssigen Ammoniak.

Als Katalysatoren verwendet man erfindungsgemäss Einsenkatalysatoren, die durch Reduktion von Eisenoxiden mit Wasserstoff bei einer Temperatur $\leq$ 500°C erhalten worden sind. Geeignete Katalysatoren werden beispielsweise in der DE-OS 2 429 293 beschrieben.

Besonders bewährt haben sich geformte metallische Eisenkatalysatoren, die aus anisometrischen Eisenoxidteilchen durch Reduktion mit Wasserstoff bei Temperaturen $\leq$ 500°C erhalten worden sind und die einen Gehalt an Gleitmitteln enthalten. Vorteilhaft weisen die metallischen Eisenteilchen einen Reduktionsgrad $\geq$ 95% auf. Unter Reduktionsgrad ist der Anteil des verfügbaren Eisens in Prozent zu verstehen, der in metallischer Form vorliegt. Bevorzugt geht man von anisometrischen $\gamma$-Eisenoxiden, insbesondere $\gamma$-Eisen-III-oxid und $\gamma$-Eisen-III-oxidhydrat aus. $\gamma$-Eisen-III-oxidhydrat, das unter dem Namen Lepidokrokit bekannt ist, wird bevorzugt verwendet. Es ist z.B. nach dem in der DE-AS 1 061 760 beschriebenen Verfahren erhältlich. Die anisometrischen Eisenoxide haben z.B. eine mittlere Teilchenlänge von 0,1 bis 2 $\mu$m, vorzugsweise 0,2 bis 1,2 $\mu$m bei einem Längen : Dickenverhältnis von 5:1 bis 40:1 eine spezifischen Oberfläche nach BET von 25 bis 28m²/g. In gleicher Weise lassen sich auch die getemperten Produkte der genannten Eisen-III-oxide verwenden. Die Temperung erfolgt zweckmässig bei 250 bis 700°C.

Die geformten Eisenkatalysatormassen enthalten zusätzlich Gleitmittel, z.B. anorganische Stoffe mit Gitterstruktur wie Talkum oder insbesondere Graphit. Vorteilhaft enthalten geformte Katalysatoren 1 bis 5 Gew.-% Gleitmittel, bezogen auf die gesamte Katalysatormasse, aus Eisenteilchen und Gleitmittel. Besonders bewährt hat sich Graphit als Gleitmittel. Die geformte Eisenkatalysatormasse besteht somit im wesentlichen aus metallischen Eisenteilchen, geringen Mengen Eisenoxid, entsprechend dem Reduktionsgrad der Eisenteilchen und einem Gleitmittel. Die geformte Eisenkatalysatormasse, z.B. in Form von Kugeln, Tabletten oder Strängen hat vorteilhaft eine Druckhärte von grösser $\geq$ 300 kp/cm². 

Die bevorzugt verwendeten geformten Eisenkatalysatormassen stellt man vorteilhaft her, indem man z.B. von $\gamma$-Eisen-III-oxiden, insbesondere $\gamma$-Eisen-III-oxidhydrat (Lepidokrokit) ausgeht. In gleicher Weise lassen sich auch die getemperten Produkte der genannten Eisen-III-oxide verwenden, wobei die Temperung zweckmässig bei 250 bis 700°C erfolgt. Eisen-III-oxidhydrat erhält man beispielsweise aus wässrigen Lösungen von Eisensalzen durch Fällen mit Natronlauge nach einem Verfahren, wie es in der DE-AS 1 061 760 beschrieben wird. Vorteilhaft wäscht man die γ-Eisenoxidhydratpartikel bis der Alkaligehalt kleiner 0,1 Gew.-%, berechnet als Natriumoxid, beträgt. Die nadelförmigen Eisen-III-oxid-Teilchen werden mittels Wasserstoff in der Wirbelschicht, im Drehrohrofen oder vorzugsweise in einem gerührten Festbett z.B. bei einer Temperatur von 260 bis 500°C, insbesondere 300 bis 450°C, innerhalb von 3 bis 36 Stunden reduziert. Vorteilhaft wendet man einen trockenen Wasserstoffstrom an, wobei man eine relativ grosse Wasserstoffströmungsgeschwindigkeit einhält. Es hat sich bewährt, wenn man mindestens einen 60fachen Wasserstoffüberschuss anwendet. Vorteilhaft reduziert man so lange, bis der Reduktionsgrad ≥ 95% betägt. So erhaltene nadelförmige, im wesentlichen aus Eisen bestehende Metallteilchen, besitzen noch weitgehend die von den Ausgangsstoffen herrührende Gestalt und sind trotz der vorangegangenen Umwandlungsreaktion einheitlich.

Anschliessend werden die so erhaltenen Metallteilchen stabilisiert. Hierunter versteht man das Umhüllen der Metallteilchen mit einer Oxidschicht durch kontrollierte Oxidation, um die durch die grosse freie Oberfläche der kleinen Teilchen bedingte Pyrophoritität zu beseitigen. Dies wird durch Überleiten eines molekularen Sauerstoff enthaltenden Gases, z.B. eines Luft-Stickstoffgemisches, unter genauer Einhaltung einer vorzugsweise 100°C, insbesondere 80°C nicht überschreitenden Temperatur, über das Metallpulver erreicht. Nach der Stabilisierung soll der Reduktionsgrad nicht unter 80%, vorzugsweise nicht unter 90% liegen. Die stabilisierten Eisenteilchen haben eine Oberfläche nach BET von 4 bis 25 $m^2/g$, vorzugsweise 8 bis 12 $m^2/g$ sowie Partikellängenvon 0,05 bis 2,0 µm, ferner Porenvolumina unterhalb 0,4 $cm^3/g$, wobei das Verhältnis der Mikrozu Makroporen zugunsten der Makroporen im Bereich von 1:6 bis 1:10 liegt.

Die so stabilisierten Eisenteilchen werden mit einem inerten Gleitmittel, vorzugsweise Graphit, vermischt. Vorteilhaft wendet man 2 bis 5 Gew.-% Gleitmittel an. Das Gemisch aus stabilisierten Eisenteilchen und Gleitmitteln wird zweckmässig unter Stickstoffabdeckung zu Formkörpern verarbeitet, z.B. zu Tabletten verpresst. Die Druckhärte der Formkörper soll ≥ 300 kp/$cm^2$ betragen. Die so erhaltenen Formkörper werden durch Behandeln mit Wasserstoff in relativ grossem Überschuss, z.B. dem 60fachen Überschuss bei einer Temperatur ≤ 500°C, vorzugsweise 300 bis 460°C bei Atmosphärendruck oder unter erhöhtem Druck, z.B. 100 bis 150 bar, aktiviert. Hierbei soll ein Reduktionsgrad von vorteilhaft ≥ 95% erreicht werden. Durch die Aktivierung steigt die Druckhärte der Formkörper z.B. von 300 auf 600 bis 800 kg/$cm^2$.

Die Hydrierung des Dinitrils kann absatzweise erfolgen, vorteilhaft führt man die Umsetzung kontinuierlich durch, z.B. in Rieselfahrweise mit fest angeordneten Eisenkatalysatoren.

Aus dem Hydriergemisch erhält man gegebenenfalls nach Verdampfen des Ammoniaks die erzeugten cyclischen Imine durch Destillation in reiner Form. 3-Methylpiperidin findet Verwendung als Vulkanisationsbeschleuniger und Zusatz von Schmiermitteln, ferner ist es ein Zwischenprodukt zur Herstellung von Nikotinsäure, die als Futtermittelzusatz von Bedeutung ist.

Das erfindungsgemässe Verfahren sei an folgenden Beispielen veranschaulicht.

*Beispiel 1*

Herstellung des Katalysators

600 kg nadelförmigen Lepidokrokits (γ-FeOOH) mit einem Chlorgehalt kleiner 0,1 Gew.-% und einem $Na_2$-Gehalt kleiner 0,1 Gew.-%, hergestellt nach der DE-AS 1 061 760 mit einer speziellen Oberfläche von 32 $m^2/g$, einer mittleren Nadellänge von 0,8 µm und einem Längen- zu Dickenverhältnis der Nadeln von 22:1, einer Schüttdichte von 0,37 $g/cm^3$ und mit einem pH-Wert von 7,2 werden in einem gerührten Festbett bei 400°C 38 Stunden mit 400 $Nm^3$ Wasserstoff zu metallischem Eisen (Fe ≥ 95%) reduziert (stöchiometrischer Wasserstoffüberschuss 64). Anschliessend wird mit einem Stickstoff-Luftgemisch das pyrophore nadelige Metallpigment bei einer Temperatur von 60°C mit einer stabilisierenden Oxidschicht überzogen, wobei der Reduktionsgrad nicht unter 90% absinken soll. Die Ausbeute beträgt 400 kg. Die Eisenteilchen haben eine spezifische Oberfläche von 7,2 $m^2/g$ (nach BET) und besitzen in der elektronenmikroskopischen Aufnahme eine anisotrope geometrische Form (nadel- bzw. stäbchenförmig). Zur Herstellung von geformten Massen mit Durchmesser 5 mm und einer Höhe von 4 mm wird das stabilisierte pulverförmige Metallpigment mit 2 Gew.-% Graphit vermischt und unter Stickstoffabdeckung tablettiert. Die Druckhärte der Tabletten soll nicht unter 300 kp/$cm^2$ liegen.

Hydrierung von 2-Methylglutarsäuredinitril

In einem Rohrreaktor von 2 m Länge und einem inneren Durchmesser von 45 mm werden 3 l eines wie vorbeschrieben erhaltenen geformten Eisenkatalysators plaziert. Die Passivierung des Katalysators wird durch 24stündige Behandlung mit Wasserstoff bei einer Temperatur von 360°C aufgehoben. In Rieselfahrweise werden dann stündlich 0,8 l 2-Methylglutarsäuredinitril, 0,8 l flüssiger Ammoniak sowie 4,6 l rohes Hydriergemisch sowie Wasserstoff zugeführt. Die Hydrierung erfolgt unter einem Druck von 260 bar und bei einer Temperatur von 230°C. Nach dem Verdampfen des Ammoniaks ergibt die gaschromatographische Analyse des rohen Hydrierproduktes 93 Gew.-% Methylpiperidin. Durch destillative Rückstandsbestimmung wurde ein Gehalt von 0,6 Gew.-% an Oligomeren des 2-Methylpentamethylendiamins ermittelt.

*Beispiel 2*

Man verwendet den in Beispiel 1 beschriebenen Rohrreaktor und Katalysator. Bei einer Reaktortemperatur von 230°C beaufschlagte man o.g. Reaktor pro Stunde mit 0,8 l 2-Methylglutarsäuredinitril und 6,4 l des dabei anfallenden rohen Hydriergemisches.

Die GC-Analyse des rohen Hydriergemisches ergab:

0,4   Gew.-% Methylpentylamin
98,7   Gew.-% 3-Methylpiperidin
0,8   Gew.-% 2-Methylpentamethylendiamin
0,03 Gew.-% 2-Methylglutarsäuredinitril

Bei der Destillation fielen 0,2% vom GC nicht erfassbare Höhersieder an, so dass sich eine Selektivität von 98,5% 3-Methylpiperidin errechnet.

*Beispiel 3*

Dem Reaktor und Katalysator gemäss Beispiel 1 wurden pro Stunde 1,2 l eines aus 90 Gew.-% 2-Methylglutarsäuredinitril und 10 Gew.-% Ethylbernsteinsäuredinitril bestehenden Dinitrilgemisches zugeführt. Unter Einhaltung einer Hydriertemperatur von 240°C und eines Wasserstoffdrucks von 260 bar wurden 7,8 l/Stunde des rohen Hydriergemisches in den Reaktor zurückgeführt.

Die GC-Analyse des rohen Hydriergemisches ergab:

3     Gew.-% Monoamine
89    Gew.-% 3-Methylpiperidin
5     Gew.-% 2-Ethylpyrrolidin
0,3   Gew.-% Diamine
0,02 Gew.-% unumgesetzte Dinitrile

Bei der Destillation fielen 0,6% von GC nicht erfassbare Höhersieder (bzw. Rückstand) an, so dass sich eine Selektivität von 98,2% 3-Methylpiperidin und 69,7% 2-Ethylpyrrolidin, entsprechend einer Gesamtselektivität von 95,3%, errechnet.

*Beispiel 4*

Dem Reaktor und Katalysator gemäss Beispiel 1 wurden pro Stunde 0,8 l Ethylbernsteinsäuredinitril zugeführt. Es wurde eine Hydriertemperatur von 190°C und ein Wasserstoffdruck von 260 bar eingehalten. 7,8 l/h des unter diesen Bedingungen anfallenden Hydriergemisches wurden in den Reaktor zurückgeführt.

GC-Analyse:

96,5   Gew.-% 2-Ethylpyrrolidin
2,3   Gew.-% 2-Ethyltetramethylendiamin
0,01  Gew.-% 2-Ethylsusuccinonitril
1,2   Gew.-% Monamine und Höhersieder

*Beispiel 5*

Dem Reaktor und Katalysator gemäss Beispiel 1 wurden bei 270°C und 260 bar $H_2$ pro Stunde 0,4 l Adipinsäuredinitril, 1,2 l Ammoniak und 2,4 l Hydriergemisch zugeführt. Azacycloheptan fiel neben 3% Bishexamethylentriamin, 7% Hexylamin und 15% Hexamethylendiamin in einer Ausbeute von 70% an.

**Patentansprüche**

1. Verfahren zur Herstellung von 5- bis 7gliedrigen cyclischen Iminen mit einer Iminogruppe und 4 bis 6 Kohlenstoffatomen im Ring, wobei mindestens ein Kohlenstoffatom einen Alkylrest mit 1 bis 4 Kohlenstoffatomen als Substituenten enthalten kann, durch Hydrieren von $C_2$- bis $C_4$-Alkandinitrilen, die an mindestens einem Kohlenstoffatom einen Alkylrest mit 1 bis 4 Kohlenstoffatomen als Substituenten haben können, bei einer Temperatur von 180 bis 300°C und unter erhöhtem Druck in flüssiger Phase in Gegenwart von Katalysatoren, dadurch gekennzeichnet, dass man Eisenkatalysatoren verwendet, die durch Reduktion von Eisenoxiden mit Wasserstoff bei einer Temperatur $\leq$ 500°C erhalten worden sind.

2. Verfahren nach Anspruch 1, dadurch gekennzeichnet, dass man 2-Methylglutarsäuredinitril verwendet.

3. Verfahren nach den Ansprüchen 1 und 2, dadurch gekennzeichnet, dass man Temperaturen von 200 bis 260°C einhält.

4. Verfahren nach den Ansprüchen 1 bis 3, dadurch gekennzeichnet, dass man Drücke von 150 bis 320 bar einhält.

5. Verfahren nach den Ansprüchen 1 bis 4, dadurch gekennzeichnet, dass man je Volumenteil frisch zugeführtem Dinitril 1 bis 15 Volumenteile rohes Hydriergemisch zurückführt.

6. Verfahren nach den Ansprüchen 1 bis 5, dadurch gekennzeichnet, dass man geformte metallischen Eisenkatalysatoren verwendet, die aus anisometrischen Eisenoxidteilchen durch Reduktion mit Wasserstoff bei einer Temperatur $\leq$ 500°C erhalten worden sind und die ein Gleitmittel enthalten.

7. Verfahren nach den Ansprüchen 1 bis 6, dadurch gekennzeichnet, dass man von anisometrischem γ-Eisen-III-oxidhydrat ausgeht.

8. Verfahren nach den Ansprüchen 1 bis 7, dadurch gekennzeichnet, dass der geformte metallische Eisenkatalysator Graphit als Gleitmittel enthält.

**Claims**

1. A process for the preparation of a 5-membered, 6-membered or 7-membered cyclic imine, which possesses one imino group and 4 to 6 carbon atoms in the ring and in which at least one carbon atom can bear alkyl of 1 to 4 carbon atoms as substituent, by hydrogenation of a $C_2$-$C_4$-alkanedinitrile, in which at least one carbon atom can bear alkyl of 1 to 4 carbon atoms as substituent, in the liquid phase, at from 180 to 300°C and under superatmospheric pressure, in the presence of a catalyst, wherein an iron catalyst which has been obtained by reduction of an iron oxide with hydrogen at $\leq$ 500°C is used.

2. A process as claimed in claim 1, wherein 2-methylglutarodinitrile is used.

3. A process as claimed in claims 1 and 2, wherein a temperature of from 200 to 260°C is maintained.

4. A process as claimed in claims 1 to 3, wherein a pressure of from 150 to 320 bar is maintained.

5. A process as claimed in claims 1 to 4, wherein from 1 to 15 parts by volume of crude hydrogenation mixture are recycled per part by volume of freshly supplied dinitrile.

6. A process as claimed in claims 1 to 5, wherein a molded metallic iron catalyst is used which has been obtained from anisometric iron oxide particles by reduction with hydrogen at $\leq$ 500°C, and contains a lubricant.

7. A process as claimed in claims 1 to 6, wherein anisometric $\gamma$-iron(III) oxide hydroxide is used as starting material.

8. A process as claimed in claims 1 to 7, wherein the molded metallic iron catalyst contains graphite as lubricant.

**Revendications**

1. Procédé de préparation d'imines cycliques de 5 à 7 termes, à un groupe imino et 4 à 6 atomes de carbone dans le noyau, au moins un atome de carbone pouvant contenir, comme substituant, un reste alkyle de 1 à 4 atomes de carbone, par hydrogénation d'alcane(en $C_2$ à $C_4$)-dinitriles, qui peuvent avoir, comme substituant, sur au moins un atome de carbone, un reste alkyle de 1 à 4 atomes de carbone, à une température de 180 à 300°C et sous pression élevée, en phase liquide, en présence de catalyseurs, caractérisé par le fait que l'on utilise des catalyseurs au fer qui ont été obtenus par réduction d'oxydes de fer avec de l'hydrogène à une température $\leq$ 500°C.

2. Procédé selon la revendication 1, caractérisé par le fait qu'on utilise du dinitrile d'acide 2-méthyl-glutarique.

3. Procédé selon les revendications 1 et 2, caractérisé par le fait que l'on maintient des températures de 200 à 260°C.

4. Procédé selon les revendications 1 à 3, caractérisé par le fait que l'on maintient des pressions de 150 à 320 bar.

5. Procédé selon les revendications 1 à 4, caractérisé par le fait que, pour chaque partie en volume de dinitrile fourni frais, on ramène 1 à 15 parties en volume de mélange d'hydrogénation brut.

6. Procédé selon les revendications 1 à 5, caractérisé par le fait qu'on utilise des catalyseurs métalliques au fer moulés, qui sont obtenus à partir de particules d'oxyde de fer anisométriques, par réduction avec de l'hydrogène, à une température de $\leq$ 500°C et qui contiennent un lubrifiant.

7. Procédé selon les revendications 1 à 6, caractérisé par le fait que l'on part d'oxyde de fer(III)-$\gamma$ hydraté.

8. Procédé selon les revendications 1 à 7, caractérisé par le fait que le catalyseur métallique au fer, moulé, contient du graphite, comme lubrifiant.